# EUROPEAN PATENT SPECIFICATION

(11) **EP 1 205 148 B1**
(45) Date of publication and mention of the grant of the patent: **08.07.2009**
(21) Application number: 01129315.6
(22) Date of filing: 23.05.1994
(51) Int. Cl.: A61B 17/12, A61F 5/00

(54) **Universal gastric band**
Universelles Magenband
Ceinture gastrique universelle

(30) Priority: 27.05.1993 US 68411
(43) Date of publication of application: 15.05.2002
(62) Divisional of application: 94918102.8
(73) Proprietor: ALLERGAN, INC., Irvine, CA 92612 (US)
(72) Inventor: Vincent, Vernont L., Santa Barbara, CA 93110 (US)
(74) Representative: HOFFMANN EITLE

(56) References cited:
- EP-A- 0 611 561
- WO-A-86/04498
- DE-U- 9 014 048
- US-A- 5 074 868
- US-A- 5 152 770
- US-A- 5 226 429

## Description

### BACKGROUND OF THE INVENTION

### 1. Field of the Invention

This invention generally relates to devices for the control of obesity, and, more particularly, to a surgically implantable adjustable band for encircling the stomach. Claim 1 further below is directed to a gastric band for treatment of morbid obesity.

### 2. Prior Art

Several years ago, Kuzmak, et al. in U.S. Patent 4,592,339, to which the skilled reader is referred for details, described a belt-like band for encircling the stomach to control morbid obesity. The band comprised substantially a belt which could be passed around the stomach in a circling position and then cinched tight in order to adjust the stoma opening within the stomach. Further improvements in the gastric band have included having an adjustable portion of the band comprising an inflatable member which permits the fine adjustment of the stoma opening after the size of the stoma is initially set by the band tightening procedure. The band tightening procedure normally involves the placement of a calibrating apparatus in the stomach to detect the stoma size. Once the apparatus is placed within the stomach the gastric band is cinched down tight until the stoma opening approximates the desired size. The band is fastened in position, usually by sutures, and the stoma opening finally adjusted by injecting a fluid into an inflatable member which is coextensive with a portion of the inner stomach-contacting surface of the band. The means for injecting the fluid into the inflatable member usually comprises a fill port located beneath the skin which can be accessed extracorporeally by transdermal injection. Thus, following implantation, the gastric band can be adjusted, within a narrow range, to enlarge or reduce the stoma as required.

One of the disadvantages of the prior art gastric bands is the difficulty in tensioning the band around the stomach to approximate the desired stoma. This is particularly difficult when the band is to be placed laparoscopically. When the band is placed laparoscopically, special instruments are required to tension the band prior to the fine adjustment of the stoma. Such instruments as are required to grasp the band and pull it into an encircling position around the stomach then cinch it tight, are difficult to manipulate through a laparoscopic canula. It would therefore be desirable to provide a band having an inflatable member thereon which can be easily fastened into an encircling position around the stomach and in which the adjustment of the stoma opening can be regulated solely by the injection of fluid into the inflatable member.

US 5,152,770, which the EPO found to represent the closest prior art (see T 1240/05), was found to disclose an implantable device for occluding the orifice of the stomach in obese patients. The gastric band of US 5,152,770 comprises a body portion having a head end and a tail end and an inner stomach-facing surface therebetween, the tail end comprising an elongate tubular member capable of fluid tight connection to an injection reservoir and the tubular member being in fluid communication with an inflatable member provided on the inner surface of the body portion. The head end comprises a perforation or slot for receiving the tail end and for locking the band around the stomach of a patient. When using the device, the tubular member is passed through the perforation and the tail end or locking means is forced through the perforation to ensure reliable and safe locking.

The gastric band of US 5,152,770, however, is not adapted for laparoscopic placement around the stomach of a patient since such placement requires the use of laparoscopic cannula (trocar) to introduce the band and other special instruments in order to attach the band around the stomach. Having regard to the width of the elongate strip portion or plate of the device of US 5,152,770, the known band could hardly have been introduced laparoscopically into an incision in the abdomen of a patient but, instead, only by laparotomy. Further, once the device of US 5,152,770 has been locked in place, the band cannot form an inner circumference having a continuous circle since the complementary locking elements are presented at a right angle to each other. Furthermore, the inflatable member of US 5,152,770 is not coextensive with the inner surface of the body portion, it covers only a part of the band surface.

Further prior art is known from US 4,632,114, US 5,074,868 and WO 86/04498 Al

### SUMMARY OF THE INVENTION

An object of this invention is to provide a gastric band which can be laparoscopically placed and non-adjustably fastened into an encircling position around the stomach to form a ring having an inner diameter.

It is yet another object of this invention to provide a gastric band wherein the band can be easily affixed in an encircling position around the stomach by conventional laparoscopic instruments.

It is yet another object of this invention to provide a gastric band adapted for laparoscopic placement around the stomach and thereafter to be non-adjustably locked into a circle around the stomach to compress the encircled portion of the stomach to constrict the stoma thereof which constriction may be further adjusted solely by means of an inflatable member.

It is yet a further object of the invention to provide a gastric band having an inflatable member deployed on an inner surface wherein said inner surface presents a smooth, continuous surface when the band is fastened in an encircling position and the inflatable member is inflated.

To this end, the invention provides a gastric band as recited in claim 1. Preferred embodiments are recited in the dependent claims.

The invention will now be detailed with reference to the preferred embodiments.

### BRIEF DESCRIPTION OF THE DRAWINGS

Figure 1 is a perspective view of the gastric band according to the present invention.
Figure 2 is the gastric band according to the present invention fastened in an encircling position and partially inflated.

### DESCRIPTION OF THE PREFERRED EMBODIMENT

As mentioned earlier, prior art gastric bands have two steps or stages of adjustment: the first step roughly affixes the gastric band in a position to encircle the stomach with the stoma opening approximating the desired size. This first adjustment is analogous to tightening a belt around the waist until the buckle tongue passes through one of a plurality of holes rendering comfortable tension. The next step in the adjustment requires injection of a fluid into the inflatable member to bring the stoma opening to the desired size.

Prior art gastric bands, lacking a large range of inflatable adjustment, require an initial "rough" adjustment. The "rough" adjustment comprising the first step in the gastric stoma-constricting process requires tensioning the prior art band, and holding the tension on the band, while sutures are placed to lock the band in the encircling position. This is a cumbersome procedure to perform laparoscopically and the design of the present invention overcomes some of the difficulties with tensioning the band.

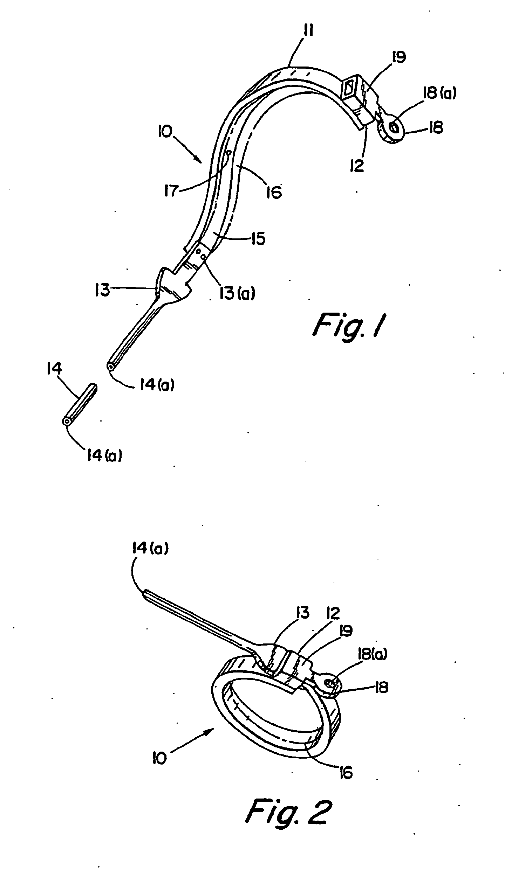

Turning now to Figure 1, we see a gastric band, generally indicated at the numeral 10, which has a body portion 11 with an inner stomach-facing surface 15. The body portion 11 has a head end 12 and a tail end 13 with one or more suture holes 13(a) therein. A fill tube 14, which is generally a tube having a single lumen 14(a) coextensive therewith, is in fluid communication with an inflatable member 16 on the inner surface 15 of the band body 11. It is an important feature of this invention that the inflatable portion 16 is substantially co-extensive with the inner surface 15 of the body portion 11 so as to present a substantially continuous surface free of abrupt contours when the band encircles the stomach and the inflatable portion 16 is inflated. The central lumen 14(a) of the fill tube 14 enters into the inflatable member 16 at lumen opening 17. The head 12 of the body portion 11 has a buckle 19 with a pull tab 18 having a suture hole 18(a) integral therewith. Both portions which receive a suture, that is the pull tab 18 and the tail 13, are preferably medical grade silicone reinforced with dacron.

In practice, the gastric band is placed in the circling position around the stomach as shown in Figure 2. (In Figure 2 the stomach is omitted for clarity.) This is accomplished by pushing the fill tube 14 through a laparoscopic canula (not shown) in the patient's abdomen. Laparoscopic placement consists of blunt dissection around the greater curvature of the stomach. The end of the fill tube 14 is passed around the stomach, and the tail 13 is attached to the buckle 19, so that the buckle and the tail are non-adjustably and irreversibly affixed to one another. In this sense, the band is a "one-size-fits-all" device. That is, for a particular band there is only one single position in which the tail and buckle can be attached to one another. Further adjustment of the stoma; the narrow opening in the stomach created by the band, is performed solely by inflation or deflation of the inflatable member 16 after the band is secured in this single position.

Prior art gastric bands employ an adjustable balloon portion used for small post-operative adjustment of the stoma if necessary. The laparoscopic hydraulic gastric band outlined herein has a larger balloon covering a greater area of the inner surface of the gastric band than taught in the prior art. The inflatable member or balloon 16 preferably is coextensive with the inner stomach-facing surface 15 of the band between the head end 12 and the tail end 13. This larger balloon 16 is utilized to adjust the stoma at the time of surgery. The interior of the adjustable balloon 16 is in fluid communication with an injection reservoir (not shown) by means of the central lumen 14(a) of the fill tube 14, as with prior art adjustable gastric bands. The inflatable member 16 is gradually inflated with saline via the injection reservoir (not shown) such that the inflatable member 16, which, as stated above, is coextensive with the inner surface 15 of the band 11 between the head end 12 and the tail end 13, presses on and constricts the stomach wall underlying the band. This results in the decrease of the opening (stoma) inside the stomach directly under the encircling band. The continuous surface presented by the enlarged inflatable member to the underlying tissue inhibits squeezing of the tissue into wrinkles, dimples or similar discontinuities such as are present on the inner surface of prior art bands and thereby prevents necrosis.

An electronic pressure transducer known as a gastrostenometer electronic sensor, coupled to a calibration probe known generically as a "calibration tube" is used to measure the size of the stoma. The calibration tube is introduced through the patient's mouth and esophagus into the stomach. The band is inflated, tightening it around the tip of the calibration tube. The gastrostenometer electronic sensor indicates the stoma diameter created as the band is tightened.

The present invention allows for the laparoscopic implantation of a therapeutic surgical device without the risks associated with major abdominal incisions in the obese patient. Faster healing, reduced complications, reduced hospital stay and drastically reduced pain are among the advantages of laparoscopic placement. The gastric band according to the present invention facilitates laparoscopic placement and will also allow wider use of the gastric band system to create a gastric restricter procedure for the treatment of morbid obesity. This band enables more surgeons, skilled in the art of laparoscopic surgery to undertake this operation without the need for highly specialized, difficult to use tensioning instruments. Since there is only one single, non-adjustable locking position for the band around the stomach, the procedure itself should lend itself to more rigorous standardization. Also, more patients will consider having this procedure performed because of the advantages of laparoscopic surgery over traditional abdominal laparotomies. These advantages, coupled with the advantages of the prior art gastric bands, which enable post operative fine tuning of the stoma, enable a more facile procedure to be performed.

In general, the smoother or more continuous the inner surface 15 of the band 10, the less likely the chance of necrosis. The single locking or fastening position of the present gastric band ensures that the inflatable member 16 will be substantially coextensive with the inner surface 15 of the band 10 and continuous with the buckle 19. This fixed-diameter band enables the inner surface 15 to present as a continuous surface to the encircled tissue as the band is inflated, thereby preventing necrosis. Prior art gastric bands lack a provision for assuring that the inflatable member is adjacent to and continuous with the buckle portion when the band is locked into an encircling position. This permits a gap or space between the inflatable member and the buckle along the inner surface when the inflatable member is inflated. Tissue can squeeze into such a gap, causing necrosis. The reduction and/or elimination of such discontinuities on the inner surface of the (inflated) band as taught hereinabove reduces the possibility of necrosis.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A gastric band (10) for treatment of morbid obesity, comprising a body portion (11) having a head end (12) and a tail end (13) and an inner stomach-facing surface (15) therebetween,
said tail end (13) comprising an elongated tubular member (14) capable of fluid tight connection to an injection reservoir,
said head end (12) comprising thereon a buckle (19) for receiving said tail end (13) and for locking said gastric band (10) non-adjustably into a circle having an inner circumference,
an inflatable member (16) on the inner surface (15) being in fluid communication with said tubular member (14), the inflatable member (16) being both
substantially coextensive with said inner surface (15) of said body portion (11) and continuous with said buckle (19) when said gastric band (10) is non-adjustably locked into said circle,
said gastric band (10) being adapted for laparoscopic placement around the stomach of a patient.

2. The gastric band of claim 1, wherein said inflatable member (16) does not wrinkle or fold, thereby presenting a substantially smooth contour along said inner circumference.

3. The gastric band of claim 1 or 2, wherein the tail end (13) comprises one or more suture holes (13a) therein.

4. The gastric band of any of the preceding claims, wherein the buckle (19) comprises a pull tab (18) having a suture hole (18a) integral therewith.

5. The gastric band of any of the preceding claims, wherein the elongate tubular member (14) comprises a single lumen (14a) which is coextensive with the elongate tubular member (14).

6. The gastric band of claim 5, wherein the central lumen (14a) of the elongated tubular member (14) enters into the inflatable member (16) at a lumen opening (17).

7. The gastric band of claims 3 and 4, or claims 3 and 4 and any of claims 5 and 6, wherein the pull tab (18) and the tail end (13) are medical grade silicone reinforced with Dacron^{®}.

## Patentansprüche

1. Magenband (10) zur Behandlung von morbider Fettleibigkeit, umfassend einen Körperabschnitt (11) mit einem Kopfende (12) und einem Schwanzende (13) und dazwischen einer dem Magen zugewandten Innenfläche (15), wobei
das Schwanzende (13) ein längliches, röhrenförmiges Element (14) mit der Fähigkeit zu einer fluiddichten Verbindung mit einem Injektionsreservoir aufweist,
das Kopfende (12) auf ihm eine Schnalle (19) zum Aufnehmen des Schwanzendes (13) und zum nichtverstellbaren Schließen des Magenbandes (10) zu einem Kreis mit einem Innenumfang aufweist,
ein aufblasbares Element (16) auf der Innenfläche (15) mit dem röhrenförmigen Element (14) in Fluidkommunikation ist, das aufblasbare Element (16) sowohl mit der Innenfläche (15) des Körperabschnitts (11) koextensiv als auch kontinuierlich mit der Schnalle (19) ist, wenn das Magenband (10) nichtverstellbar zu dem Kreis geschlossen ist,
das Magenband (10) zur laparoskopischen Anordnung um den Magen eines Patienten ausgebildet ist.

2. Magenband (10) nach Anspruch 1, wobei das aufblasbare Element (16) sich nicht knittert oder faltet, wodurch eine im Wesentlichen glatte Kontur entlang des Innenumfangs bereitgestellt wird.

3. Magenband nach Anspruch 1 oder 2, bei dem das Schwanzende (13) eines oder mehrere Nahtlöcher (13a) darin aufweist.

4. Magenband nach einem der vorhergehenden Ansprüche, bei dem die Schnalle (19) eine Ziehschlaufe (18) mit einem Nahtloch (18a) aufweist.

5. Magenband nach einem der vorhergehenden Ansprüche, bei dem das längliche, röhrenförmige Element (14) eine einziges Lumen (14a), welches mit dem länglichen, röhrenförmigen Element (14) koextensiv ist, aufweist.

6. Magenband nach Anspruch 5, bei dem das zentrale Lumen (14a) des länglichen, röhrenförmigen Elements (14) in das aufblasbare Element an einer Lumenöffnung (17) eindringt.

7. Magenband nach Anspruch 3 und 4, oder Anspruch 3 und 4 und einem der Ansprüche 5 und 6, bei dem die Ziehschlaufe (18) und das Schwanzende (13) aus Silikon medizinischer Güteklasse verstärkt mit Dacron® sind.

## Revendications

1. Anneau gastrique (10) pour le traitement de l'obésité morbide, comprenant une partie corporelle (11) ayant une extrémité avant (12) et une extrémité arrière (13) et une surface interne faisant face à l'estomac (15) entre elles,
ladite extrémité arrière (13) comprenant un élément tubulaire allongé (14) capable d'être relié de façon étanche aux fluides à un réservoir d'injection,
ladite extrémité avant (12) comprenant sur celle-ci, une boucle (19) pour recevoir ladite extrémité arrière (13) et pour bloquer ledit anneau gastrique (10) de façon non ajustable dans un cercle ayant une circonférence interne,
un élément gonflable (16) sur la surface interne (15) en communication de fluide avec ledit élément tubulaire (14), l'élément gonflable (16) étant à la fois co-étendu substantiellement avec ladite surface interne (15) de ladite partie corporelle (11) et continue avec ladite boucle (19) lorsque ledit anneau gastrique (10) est bloqué de façon non ajustable dans ledit cercle,
ledit anneau gastrique (10) étant adapté pour un placement laparoscopique autour de l'estomac d'un patient.

2. Anneau gastrique selon la revendication 1, dans lequel ledit élément gonflable (16) ne se plisse pas ou ne se replie pas, présentant ainsi un contour substantiellement lisse le long de ladite circonférence interne.

3. Anneau gastrique selon la revendication 1 ou 2, dans lequel l'extrémité arrière (13) comprend un ou plusieurs trous de suture (13a).

4. Anneau gastrique selon l'une quelconque des revendications précédentes, la boucle (19) comprenant une languette de traction (18) ayant un trou de suture (18a) faisant partie intégrante de celle-ci.

5. Anneau gastrique selon l'une quelconque des revendications précédentes, dans lequel l'élément tubulaire allongé (14) comprend un lumen unique (14a) qui est co-étendu avec l'élément tubulaire allongé (14).

6. Anneau gastrique selon la revendication 5, dans lequel le lumen central (14a) de l'élément tubulaire allongé (14) entre dans l'élément gonflable (16) au niveau d'une ouverture (17) du lumen.

7. Anneau gastrique selon les revendications 3 et 4, ou les revendications 3 et 4 et l'une quelconque des revendications 5 et 6, dans lequel la languette de traction (18) et l'extrémité arrière (13) sont en silicone de qualité médicale renforcée avec du Dacron®.
